(19) Europäisches Patentamt
European Patent Office
Office européen des brevets

(11) Publication number: 0 003 863
B1

(12) # EUROPEAN PATENT SPECIFICATION

(45) Date of publication of patent specification: 22.07.81

(21) Application number: 79200088.7

(22) Date of filing: 21.02.79

(51) Int. Cl.³: C 07 J 31/00,
C 07 J 71/00,
A 61 K 31/565

(54) **Novel androstadienes, processes for their preparation and pharmaceutical compositions containing them.**

(30) Priority: 27.02.78 US 881217

(43) Date of publication of application:
05.09.79 Bulletin 79/18

(45) Publication of the grant of the European patent:
22.07.81 Bulletin 81/29

(84) Designated Contracting States:
BE CH DE FR GB IT NL SE

(56) References cited:
US - A - 2 806 041
US - A - 2 999 101

(73) Proprietor: SCHERICO LTD.
Töpferstrasse 5
CH-6004 Lucerne (CH)

(72) Inventor: Green, Michael J.
33 Wheeler Road
Kendall Park New Jersey 08824 (US)
Inventor: Tiberi, Robert
3 Woodview Drive
Englishtown New Jersey 07726 (US)

(74) Representative: Antony, Fritz, Dr. et al,
P.O. Box 601 Winkelriedstrasse 35
CH-6002 Lucerne (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Novel androstadienes, processes for their preparation and pharmaceutical compositions containing them

This invention relates to novel androstadienes having valuable pharmacological properties, to processes for their preparation and to pharmaceutical compositions containing them; and also to related androstadienes useful as intermediates in their preparation.

According to the invention we provide steroids of the formula

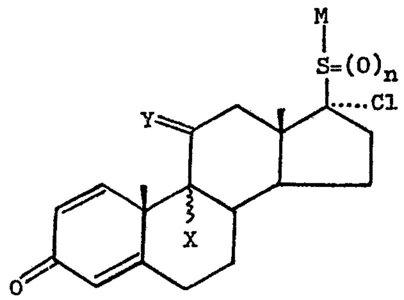

I

wherein M is a benzyl, phenethyl, methylbenzyl, dimethylbenzyl, chlorobenzyl or dichlorobenzyl group or an alkyl group having from 1 to 8 carbon atoms,
X is an $\alpha$-hydrogen, $\alpha$-fluorine or $\alpha$-bromine atom,
Y is (H,$\beta$—OH), or, provided that X is an $\alpha$-hydrogen or $\alpha$-fluorine atom, Y can also be an oxygen atom, or, provided that X is an $\alpha$-hydrogen atom, Y can also be (H,H), or X and Y together are a further 9,11 bond or a 9$\beta$,11$\beta$-epoxy group, together with an 11-hydrogen atom,
and *n* is 1, or, when Y is an oxygen atom or (H,H), *n* can also be 2.

Preferred compounds of the present invention are those of the formula

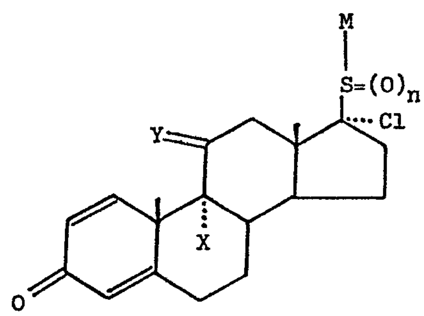

IA

wherein M is as defined above,
X is an $\alpha$-hydrogen or $\alpha$-fluorine atom,
Y is an oxygen atom, or, provided that X is an $\alpha$-hydrogen atom, Y can also be (H,H),
and *n* is 1 or 2,
since these have utility in the treatment of dermatological disorders, especially of acne vulgaris.

When M in the compounds of the formula I and IA is methylbenzyl, dimethylbenzyl, chlorobenzyl or dichlorobenzyl, the substituents methyl, dimethyl, chloro or dichloro can be in any positions on the benzene ring. Furthermore, alkyl groups included in the definition of M may be straight or branched chain, e.g., lower alkyl groups containing 1 to 5 carbon atoms such as methyl, ethyl, propyl, isopropyl, butyl, *t*-butyl and pentyl groups and higher homologs such as hexyl, heptyl and octyl groups.

Particularly preferred compounds of our invention wherein *n* is 1 are those of the formula

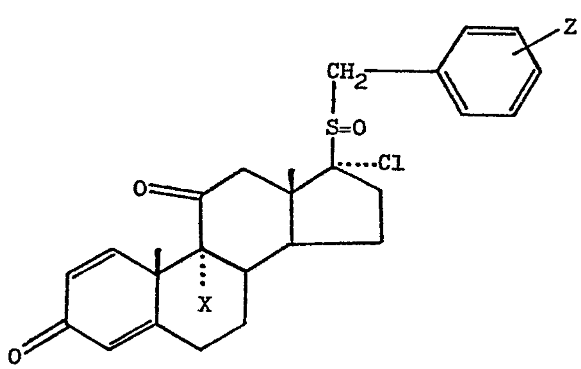

II

wherein X is as defined for formula IA and Z is a hydrogen atom or one or two methyl groups or one or two chlorine atoms.

Especially preferred compounds of this formula are:
17α-chloro-17β-[R]-benzylsulfinyl-1,4-androstadiene-3,11-dione,
17α-chloro-17β-(4-chlorobenzylsulfinyl)-1,4-androstadiene-3,11-dione,
17α-chloro-17β-(2,4-dichlorobenzylsulfinyl)-1,4-androstadiene-3,11-dione,
17α-chloro-17β-(3,4-dichlorobenzylsulfinyl)-1,4-androstadiene-3,11-dione,
17α-chloro-17β-(2-methylbenzylsulfinyl)-1,4-androstadiene-3,11-dione, and
9α-fluoro-17α-chloro-17β-[R]-benzylsulfinyl-1,4-androstadiene-3,11-dione.

Preferred compounds of this invention wherein *n* is 2 are those of the formula

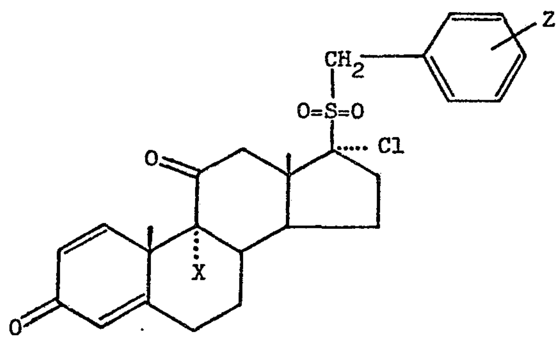

III

wherein X and Z are as defined in Formula II. A particularly preferred compound of this formula is 17α-chloro-17β-benzylsulfonyl-1,4-androstadiene-3,11-dione.

When *n* is 1 in the compounds of the formula I or IA, there is an asymmetric centre present at the sulfur atom. Therefore, these compounds can exist in the *R,S*-form, in the *R*-form, in the *S*-form, or as a mixture thereof. When X-ray crystallographic techniques have confirmed the presence of specific stereochemistry, we have so indicated in the nomeclature of the compounds.

The compounds of this invention are usually white to off-white crystalline solids. They are insoluble in water but soluble in most organic solvents, particularly in acetone, dioxan, dimethylformamide, and dimethylsulfoxide, although of limited solubility in non-polar solvents such as dialkylethers and alkane hydrocarbons.

The compounds of the formulae IA, II and III inhibit sebaceous gland activity and are therefore useful as anti-acne agents, especially by the topical or oral route.

The compounds of formula I can be prepared by standard processes. A particularly preferred process includes the step of reacting a 17α-(M-thio)-1,4-androstadiene-3-one, in which M is as defined above, with a chlorine source to introduce a 17α-chlorine atom (with inversion of the configuration of the original 17-substituent) and to oxidise the sulfur atom to sulfinyl.

According to the invention, therefore, we provide a process for the preparation of a 17α-chloro-17β-(M-sulfinyl)-1,4-androstadiene-3-one having the following partial formula at C—17

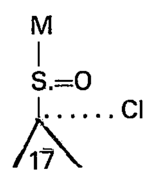

wherein M is as defined above, which comprises reacting a 17α-(M-thio)-1,4-androstadiene-3-one having the following partial formula at C—17

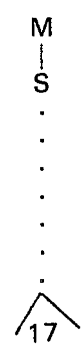

wherein M is as defined above, with a chlorine source in a solvent system comprising water and a water-soluble base, at a temperature from about −78°C to about 25°C.

The chlorine source is preferably chlorine gas or a "positive" chlorine source, e.g. N-chlorosuccinimide, 1-chlorobenzotriazole, and especially iodobenzene dichloride. At least two equivalents of the chlorine source are necessary for complete reaction of the 17α-(M-thio)-1,4-androstadiene-3-one; 2 to 5 equivalents can conveniently be used, but about 3 equivalents are preferred since this quantity generally provides best yields of the desired 17α-chloro-17β-(M-sulfinyl)-1,4-androstadiene-3-one. More than 5 equivalents of chlorine source should preferably not be used lest the steroid starting material be chlorinated at other positions also.

The solvent system is preferably water and a water-soluble base. If, however solubility problems are encountered, watermiscible organic solvents, e.g. tetrahydrofuran and dioxan, may be added. Nitrogenous bases, e.g. pyridine, trialkylamines (especially triethylamine) and collidine are preferred water-soluble bases, but inorganic water-soluble bases such as sodium hydroxide, sodium methoxide and calcium hydroxide can also be used. A minimum of 3 equivalents of base should be used to provide a good yield of desired product; an excess of base is not deleterious to the reaction.

The reaction can be carried out over a period of from 12 to 60 hours, but is usually complete in 18 to 36 hours. The temperature range is preferably from about −40°C to about 0°C and usually at around −40°C. Prolonged reaction times at higher temperatures can produce excessive chlorination of the steroid molecule.

In a preferred embodiment of our process, a 1,4-androstadiene-3-one having a 17α-thio function and in particular having the formula

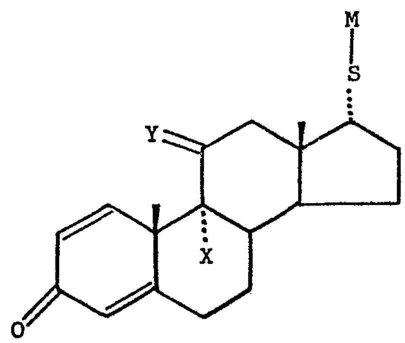

IV

wherein M is as defined above,
X is a hydrogen or fluorine atom,
and Y is an oxygen atom, or, provided that X is an α-hydrogen atom, Y can also be (H,H),
e.g., 17α-benzylthio-1,4-androstadiene-3,11-dione is dissolved in a mixture of water and pyridine (e.g., 20% aqueous pyridine), cooled to about −40°C and treated with about 3 equivalents of iodobenzene dichloride as chlorine source. The reaction is preferably followed by thin layer chromatography to ascertain its conclusion, i.e., the absence of starting compound. The 17α-chloro-17β-sulfinyl-1,4-androstadiene-3-one, in particular of the formula

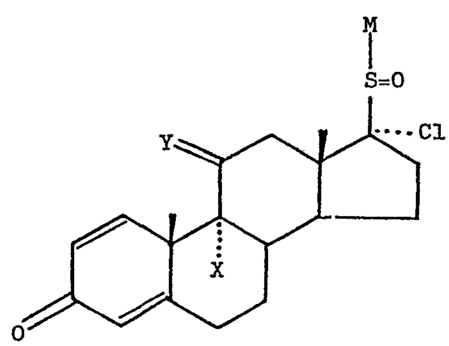

IB

wherein M, X and Y are as defined for formula IV, e.g., 17α-chloro-17β-[R]-benzylsulfinyl-1,4-androstadiene-3,11-dione, is then isolated by standard techniques.

The 17α-(M-thio)-1,4-androstadiene-3-ones used as starting compounds in the process described hereinabove, although not specifically described in the literature, can be prepared by known chemical reactions. That is, nucleophilic substitution reactions by sulfides on leaving groups at $C_3$, $C_4$ and $C_6$ are known in the literature, and analogous procedures may be applied to effect displacement by sulfide at $C_{17}$. In one such procedure, an alkali metal, e.g. sodium or potassium, is dissolved in a lower alkanol to which is added a mercaptan MSH (wherein M is as defined for formula I), e.g., benzyl mercaptan, and a 1,4-androstadiene-3-one (or -3,11-dione) having a reactive ester grouping at the

17β-position, e.g. a 17β-hydrocarbonsulfonyloxy group such as 17β-methanesulfonyloxy. The mercaptan should be present in at least one mole excess relative to the steroid; we generally prefer to use a ten-fold excess. After an appropriate reaction time (e.g. 3 days at reflux), the resulting 17α-(M-thio)-1,4-androstadiene-3-one is isolated by standard techniques. In this reaction, the lower alkanol may for example be methanol, ethanol or t-butanol, and may if desired be admixed with a polar solvent (e.g., dimethylsulfoxide, dimethylformamide or hexamethylphosphoric triamide) or an ether (e.g., tetrahydrofuran or dioxan). The temperature may be from about 25°C to reflux, but generally reflux temperatures are employed. The reaction is continued until thin layer chromatography of the reaction mixture indicates absence of the starting material (usually 24 to 120 hours).

A compound of the formula IA wherein X is a fluorine atom and Y is an oxygen atom is preferably prepared from a compound of the formula IA wherein X is a hydrogen atom and Y is an oxygen atom by the following sequence of reactions:

a) Reduction of the 11-keto group to 11β-hydroxy;
b) Dehydration to introduce a 9(11)-double bond;
c) Reaction of the 1,4,9(11)-androstatriene from step b) with a bromonium source and a strong acid in an aqueous organic solvent to yield the corresponding 9α-bromo-11β-hydroxy-1,4-androstadiene;
d) 9,11-Dehydrohalogenation to yield the 9β,11β-epoxide;
e) Reaction of this epoxide with hydrofluoric acid to yield the corresponding 9α-fluoro-11β-hydroxy-1,4 androstadiene;
f) Oxidation at the 11-position to yield the desired 11-ketone.

All these steps can be carried out under standard conditions:

a) Reduction of the 11-keto group can be effected with sodium, potassium or lithium borohydride, tetra-*n*-butylamonium borohydride, or lithium tri-*t*-butoxy-aluminium hydride, in an organic solvent, preferably sodium borohydride in an organic solvent comprising methanol or dimethylformamide.
b) The dehydration can be effected with methanesulfonyl chloride and a tertiary amine (e.g. collidine) in an dialkylamide (e.g. dimethylformamide) in the presence of sulfur dioxide.
c) The bromonium source is preferably an N-bromoamide, e.g. *N*-bromo-succinimide, and the strong acid is preferably a strong mineral acid, especially perchloric acid. The aqueous organic solvent is preferably moist dioxan or tetrahydrofuran.
d) The dehydrohalogenation is preferably effected by means of a mild base in an inert organic solvent, e.g. potassium acetate in methanol.
e) This step is preferably effected with concentrated aqueous (e.g. 70%) hydrofluoric acid.
f) The oxidation of the 11β-hydroxy group can be effected by means of chromium trioxide in pyridine but preferably by means of pyridinium chlorochromate in an inert organic solvent e.g. methylene chloride, desirably in the presence of a weak base e.g. sodium acetate.

These steps do not destroy the stereochemical integrity of the sulfur atom.

A compound of the formula I or IA in which *n* is 1 (whether produced directly by the process according to the invention or additionally by steps a) to f) above) can be oxidized to the analogous compound in which *n* is 2 by means of a peracid (e.g. m-chloroperbenzoic acid or peracetic acid) or hydrogen peroxide in an inert organic solvent, e.g., a hydrocarbon, especially an aromatic hydrocarbon, a halohydrocarbon or a lower alkanoic acid, in particular by means of *m*-chloroperbenzoic acid in benzene at about room temperature.

In the preparation of a compound of the formula IA in which X is an α-fluorine atom and *n* is 2, the above step of oxidation of the sulfinyl group preferably follows the steps a) to f) for introduction of the of the 9α-fluorine atom.

Compounds of the formula

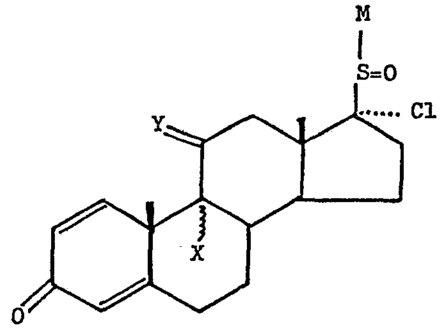

IC

wherein M is as defined for formula I, X is an α-hydrogen, α-fluorine or α-bromine atom and Y is (H,β—OH), or X and Y together form a further 9,11-bond or a 9β,11β-epoxy group, together with an 11-hydrogen atom, are useful intermediates and form a further feature of the invention. Preferred intermediates of this formula include:

17α-chloro-17β-[R]-benzylsulfinyl-11β-hydroxy-1,4-androstadiene-3-one,

17α-chloro-17β-[R]-benzylsulfinyl-11β-hydroxy-1,4,9(11)-androstatriene-3-one,
9α-bromo-17α-chloro-17β-[R]-benzylsulfinyl-11β-hydroxy-1,4-androstadiene-3-one,
9β,11β-epoxy-17α-chloro-17β-[R]-benzylsulfinyl-1,4-androstadiene-3-one, and
9α-fluoro-17α-chloro-17β-[R]-benzylsulfinyl-11β-hydroxy-1,4-androstadiene-3-one.

The following preparations show how the starting materials of formula IV can be obtained; Examples 1 and 2 illustrate but do not limit the invention:

PREPARATIONS: SULFIDE DISPLACEMENT AT CARBON-17

A. *17α-Benzylthio-1,4-androstadiene-3,11-dione*

To a solution of 13.8 gm of sodium metal in 1040 ml of ethanol are added 47 ml of benzyl mercaptan and then 21 gm of 17β-methanesulfonyloxy-1,4-androstadiene-3,11-dione. Reflux for 72 hours, then cool and filter off insoluble material. Concentrate the filtrate *in vacuo* to about 150 ml and precipitate into 1500 ml of water containing 15 ml of a 5% aqueous solution of sodium hypochlorite. Extract three times with 500 ml portions of chloroform which is then washed twice with water. Dry the chloroform extracts over anhydrous sodium sulfate, then concentrate *in vacuo* to dryness. Dissolve the residue in a minimum amount of chloroform/ethyl acetate (1:1) and pass through 300 gm of silica gel. Take 1 litre fractions using ethyl acetate/chloroform/hexane (1:10:10) as eluant. Combine desired fractions as determined by thin layer chromatography and evaporate to dryness. Recrystallize the resulting product from ethyl acetate/hexane to obtain the title compound; m.p. 134—136°C; $\lambda_{max}^{MeOH}$ 237 nm ($\varepsilon = 17,100$); $[\alpha]_D^{26}$ + 152.5° (DMF).

B. *17α-Phenethylthio-1,4-androstadiene-3,11-dione*

To a solution of 2.25 gm of sodium metal in 180 ml of ethanol add 8 ml of phenethyl mercaptan and then 3 gm of 17β-methane-sulfonyloxy-1,4-androstadiene-3,11-dione. Heat the reaction mixture at reflux for 48 hours. Obtain a residue in a manner similar to that described in Preparation A. Chromatograph the residue on a 300 gm silica gel column, eluting with chloroform/ethyl acetate (2:1). Combine the desired fractions as determined by thin layer chromatography, evaporate them and recrystallize the resulting residue from ether/hexane to obtain the title compound; m.p. 112—115°C.

C. *17α-(1-Pentylthio)-1,4-androstadiene-3,11-dione*

To a solution of 3.75 gm of sodium metal in 300 ml of ethanol are added 15 ml of n-pentylmercaptan followed by 5 gm of 17β-methanesulfonyloxy-1,4-androstadiene-3,11-dione. Reflux for 72 hours; then obtain a residue in a manner similar to that described in Preparation A. Chromatograph the residue on a 500 gm silica gel column eluting with chloroform/ethyl acetate (9:1). In a manner similar to that described in Preparation A, isolate the product and recrystallize it from ether/hexane to obtain the title compound.

D. *17α-Methylthio-1,4-androstadiene-3,11-dione*

To a solution of 6.9 gm of sodium metal in 225 ml of ethanol are added 30 ml of methyl-mercaptan and then 10 gm of 17β-methansulfonyloxy-1,4-androstadiene-3,11-dione. Reflux for 18 hours. Cool the solution to 0°C, filter off the solids and recrystallize from chloroform/hexane to obtain the title compound; m.p. 180—183°C; $[\alpha]_D^{26}$ + 101.6° (DMF).

E. *17α-(p-Chlorobenzylthio)-1,4-androstadiene-3,11-dione*

To a solution of 1.38 gm of sodium metal in 120 ml of ethanol add 6 ml of *p*-chlorobenzyl-mercaptan and then 2.26 gm of 17β-methanesulfonyloxy-1,4-androstadiene-3,11-dione. Reflux for 96 hours; then obtain a residue in a manner similar to that described in Preparation A. Dissolve the residue in a minimum amount of chloroform and pass through a 200 gm silica gel column. Elute with chloroform following the fractions by thin layer chromatography. Combine like fractions, evaporate to a residue and recrystallize it from ethyl acetate/hexane to obtain the title compound; m.p. 176—180°C.

F. *17α-(2,4-Dichlorobenzylthio)-1,4-androstadiene-3,11-dione*

To a solution of 1.04 gm of sodium metal in 80 ml of ethanol add 5.55 ml of 2,4-dichlorobenzylmercaptan and then 1.5 gm of 17β-methanesulfonyloxy-1,4-androstadiene-3,11-dione. Reflux for 76 hours under an atmosphere of nitrogen. In a manner to that described in Preparation E, isolate the title compound.

G. *17α-(3,4-Dichlorobenzylthio)-1,4-androstadiene-3,11-dione*

In a manner similar to that described in Preparation A, 3,4-dichlorobenzylmercaptan is reacted with 17β-methanesulfonyloxy-1,4-androstadiene-3,11-dione to obtain the title compound; m.p. = 131—133°C.; $[\alpha]_D^{26}$ + 150° ($CHCl_3$); $\lambda_{max}^{MeOH}$ 228 nm ($\varepsilon$ = 26,000).

H. *17α-(2-Methylbenzylthio)-1,4-androstadiene-3,11-dione*

In a manner similar to that described in Preparation A, 2-methylbenzylmercaptan is reacted with 17β-methanesulfonyloxy1,4-androstadiene-3,11-dione to obtain the title compound.

I. *17α-Methylthio-11β-hydroxy-1,4-androstadiene-3-one*

Dissolve 4.25 gm of sodium metal in 90 ml of isopropanol and 180 ml of toluene. Saturate the solution with methyl mercaptan gas. Add 18 gm of 17β-methanesulfonyloxy-11β-hydroxy-1,4-androstadiene-3-one dissolved in 270 ml of dimethylsulfoxide. Heat the reactants for 18 hours at 110°C. Pour the reaction mixture into water and extract with ethyl acetate. Wash the ethyl acetate extracts with water, then dry over anhydrous sodium sulfate. Filter the ethyl acetate extracts, evaporate and recrystallize the residue from acetone to obtain the title compound; $[\alpha]_D^{26}$ + 48.3° (DMF); $\lambda_{max}^{MeOH}$ 243 nm ($\varepsilon$ = 15,000).

J. 17α-*Benzylthio-1,4-androstadiene-3-one*

To a solution of 2.25 gm of sodium metal in 185 ml of ethanol add 8.2 ml of benzyl mercaptan and then 3 gm of 17β-methanesulfonyloxy-1,4-androstadiene-3-one. Reflux for 48 hours. Pour the reaction mixture into 5% sodium hydroxide solution and extract with ethyl acetate. Wash the ethyl acetate extracts with water, dry over anhydrous sodium sulfate and evaporate to a residue. The residue is purified on a 300 gm silica gel column eluting with chloroform/ethyl acetate (2:1) to obtain the title compound.

## Example 1
## CHLORINATION AT CARBON-17 AND SULFUR OXIDATION

A. 17α-*Chloro-17β-[R]-benzylsulfinyl-1,4-androstadiene-3,11-dione*

1. Dissolve 8 gm of 17α-benzylthio-1,4-androstadiene-3,11-dione in 240 ml of 20% aqueous pyridine. Cool the solution to −40°C and add 16.5 gm of iodobenzene dichloride and maintain at −40°C for 18 hours. Dilute with an equal volume of chloroform and wash twice with water. Dry the organic layer over anhydrous sodium sulfate, filter and then concentrate *in vacuo* to dryness. Recrystallize the residue from acetone to obtain the title compound; $[\alpha]_D^{26}$ + 111.6° ($CHCl_3$); $\lambda_{max}^{MeOH}$ 224 nm ($\varepsilon$ = 21,200) C.D. $[\theta]_{337}^{MeOH}$+ 2,800, $[\theta]_{259}^{MeOH}$−33,000, $[\theta]_{225}^{MeOH}$+ 149,000.

2. Dissolve 1 gm of 17α-benzylthio-1,4-androstadiene-3,11-dione in 40 ml of tetrahydrofuran and 7.35 ml of 1M aqueous sodium carbonate. Cool the solution to −40°C, add 2.03 gm of iodobenzene dichloride and maintain at −40°C for 24 hours. Pour the reaction mixture into water, filter off the precipitate, wash it with water and air dry. Recrystallize the precipitate from acetone to obtain the title compound.

3. In a manner similar to the foregoing, obtain the title compound by substituting 10 ml of water and 0.413 gm of sodium methoxide for the 1M aqueous sodium carbonate.

B. *17α-Chloro-17β-phenethylsulfinyl-1,4-androstadiene-3,11-dione*

Dissolve 0.42 gm of 17α-phenethylthio-1,4-androstadiene-3,11-dione in a mixture of 6.4 ml of pyridine and 1.6 ml of water. Cool the solution to −40°C, add 0.822 gm of iodobenzene dichloride and continue stirring at −40°C for 18 hours. After 18 hours, work up the reaction mixture in a manner similar to Example 1A, recrystallizing from chloroform/hexane to obtain the title compound; m.p. = 230°C, nmr (dmso-$d_6$) $\delta$ 1.19 ($C_{13}$—$CH_3$, s), 1.45 ($C_{10}$—$CH_3$, s), 6.10 ($C_4$—H, d), 6.20 ($C_2$—H, dd), 7.66 ($C_1$—H, d), 7.27 (Ø).

C. *17α-Chloro-17β-(1-pentylsulfinyl)-1,4-androstadiene-3,11-dione*

In a manner similar to that described in Example 1A, react 0.81 gm of 17α-(1-pentylthio)-1,4-androstadiene-3,11-dione, with 1.7 gm of iodobenzene dichloride in 12.8 ml of pyridine and 3.2 ml of water. After concentration, pass the residue through a 100 gm silica gel column and elute with chloroform/ethyl acetate (4:1) Recrystallize from acetone/hexane to obtain the title compound; nmr (dmso-$d_6$) $\delta$ 1.10 ($C_{13}$—$CH_3$, s), 1.38 ($C_{10}$—$CH_3$, s), 6.05 ($C_4$—H, d), 6.15 ($C_2$—H, dd), 7.65 ($C_1$—H, d).

D. *17α-Chloro-17β-methylsulfinyl-1,4-androstadiene-3,11-dione*

In a manner similar to that described in Example 1A, react 2.4 gm of 17α-methylthio-1,4-androstadiene-3,11-dione with 5.76 gm of iodobenzene dichloride in 55.6 ml of pyridine and 14.4 ml of water. Pass the concentrated residue through a 250 gm silica gel column, eluting first with chloroform and then with ethyl acetate. Evaporate the ethyl acetate eluates and recrystallize the resulting residue from acetone/hexane to obtain the title compound; nmor ($CDCl_3$) $\delta$ 1.21 ($C_{13}$—$CH_3$, s), 1.45 ($C_{10}$—$CH_3$, s), 2.55 (S—$CH_3$, s), 6.10 ($C_4$—H, d), 6.20 ($C_2$—H, dd), 7.68 ($C_1$—H, d).

E. 17α-*Chloro-17β-(4-chlorobenzylsulfinyl)-1,4-androstadiene,3,11-dione*

In a manner similar to that described in Example 1A, react 0.4 gm of 17α-(4-chlorobenzylthio)-1,4-androstadiene-3,11-dione with 0.85 gm of iodobenzene dichloride in 8 ml of pyridine and 2 ml. of water. Isolate as in Example 1D, using a 50 gm silica gel column, and recrystallizing from chloroform/ethyl acetate/hexane to obtain the title compound; nmr (dmso-$d_6$) δ 0.83 ($C_{13}$—$CH_3$, s), 1.38 ($C_{10}$—$CH_3$, s), 4.02 ($CH_2$—Ø, dd), 6.08 ($C_4$—H, d), 6.15 ($C_2$—H, dd), 7.65 ($C_1$—H, d).7.43 (Ø, q).

F. *17α-Choro-17β-(2,4-dichlorobenzyl-sulfinyl)-1,4-androstadiene-3,11-dione*

In a manner similar to that described in Example 1A, react 0.476 gm of 17α-(2′,4′-dichlorobenzylthio)-1,4-androstadiene-3,11-dione with 0.822 gm of iodobenzene dichloride in 16 ml of pyridine and 4 ml of water. After concentrating *in vacuo*, triturate the resulting residue with ether. Recrystallize the resultin solid from methylene chloride/ether to obtain the title compound; nmr (dmso-$d_6$) δ 1.09 ($C_{13}$—$CH_3$, s), 1.39 ($C_{10}$—$CH_3$, s), 4.20 ($CH_2$Ø, s), 6.04 ($C_4$—H, s), 6.09 ($C_2$—H, dd), 7.3 ($H_{6'}$, s), 7.50 ($H_{3'}H_{5'}$, s), 7.60 ($C_1$—H, d); M.S. [M+] 524, 526, 528.

G. *17α-Chloro-17β-(3,4-dichlorobenzylsulfinyl)-1,4-androstadiene-3,11-dione*

In a manner similar to that described in Example 1A, react 0.238 gms of 17α-(3,4-dichlorobenzylthio)-1,4-androstadiene-3,11-dione with 0.411 gm of iodobenzene dichloride in 8 ml of pyridine and 2 ml of water. Purify the residue by thin layer silica gel chromatography, developing with ethyl acetate/chloroform (1:4) and eluting with ethyl acetate. Recrystallize the main band from ether to obtain the title compound; m.p. 249—251°C

H. *17α-Chloro-17β-(2-methylbenzylsulfinyl)-1,4-androstadiene-3,11-dione*

In a manner similar to that described in Example 1A, react 0.946 gm of 17α-(2-methylbenzylthio-1,4-androstadiene-3,11-dione with 1.6 gm of iodobenzene dichloride in 32 ml of pyridine and 8 ml of water. Slurry the residue with ethyl acetate/ether and filter to obtain the title compound; $[\alpha]_D^{26}$ + 135° ($CHCl_3$); $\lambda_{max}^{MeOH}$ 228 nm (ε = 22,000).

I. 17α-*Chloro-17β-methylsulfinyl-11β-hydroxy-1,4-androstadiene-3-one*

In a manner similar to that described in Example 1A, react 0.475 gm 17α-methylthio-11β-hydroxy-1,4-androstadiene-3-one with 1.17 gm of iodobenzene dichloride in 12 ml of pyridine and 3 ml of water. Purify the residue by thin layer silica gel chromatography, developing with acetone/ethyl acetate/chloroform (6:47:47), and eluting with ethyl acetate. Recrystallize from acetone/hexane to obtain the title compound; m.p. 230—233°C. (decomp.); nmr (dmso-$d_6$) δ 1.40 ($C_{10}$ and $C_{13}$—$CH_3$, s), 2.47 ($SCH_3$, s), 4.28 (11α-H, mult.), 5.94 ($C_4$—H, d), 6.16 ($C_2$—H, dd), 7.29 ($C_1$—H, d).

J. *17α-Chloro-17β-benzylsulfinyl-1,4-androstadiene-3-one*

In a manner similar to that described in Example 1A, react 0.47 gm of 17α-benzylthio-1,4-androstadiene-3-one with 0.988 gm of iodobenzene dichloride in 19.2 ml of pyridine and 4.8 ml of water. Maintain the reaction at —40°C for 72 hours. Purify the residue by thin layer silica gel chromatography, developing with chloroform/ethyl acetate (2:1) and eluting with ethyl acetate to obtain the title compound; nmr ($CDCl_3$) δ 1.23 ($C_{10}$ and $C_{13}$—$CH_3$, s), 4.03 ($CH_2$Ø, dd), 6.08 ($C_4$—H, d), 6.23 ($C_2$—H, dd), 7.05 ($C_1$—H, d), 7.35 (Ø, s).

## Example 2
## OTHER 17α-CHLORO-17β-SULFUR COMPOUNDS

A. *17α-Chloro-17β-[R]-benzylsulfinyl-11β-hydroxy-1,4-androstadiene-3-one*

Stir 1.4 gm of 17α-chloro-17β-[R]-benzylsulfinyl-1,4-androstadiene-3,11-dione in 100 ml of methanol with 0.4 gm of sodium borohydride for 15 minutes at 25°C. Acidify with 1N aqueous HCl and pour into water. Filter off the solids, wash with water and air dry. Recrystallize the solids from chloroform/hexane to obtain the title compound; nmr (dmso-$d_6$) δ 1.40 ($C_{13}$—$CH_3$, s), 1.42 ($C_{10}$—$CH_3$, s), 4.02 ($CH_2$Ø, dd), 4.30 (11α—H, mult.), 5.98 ($C_4$—H, d), 6.21 ($C_2$—H, dd), 7.35 ($C_1$—H, d), 7.40 (Ø, s).

B *17α-Chloro-17β-[R]-benzylsulfinyl-1,4,9(11)-androstatriene-3-one*

Dissolve 0.6 gm of 17α-chloro-17β-[R]-benzylsulfinyl-11β-hydroxy-1,4-androstadiene-3-one in 6 ml of dimethylformamide and 1.8 of collidine. Stir the solution for 10 minutes at 25°C with 0.6 ml of 3.5% sulfur dioxide in methanesulfonylchloride (w/v). Pour the solution into water, filter off the solids, wash with water and air dry. Recrystallize the solids from acetone to obtain the title compound: nmr (dmso-$d_6$) δ 1.11 ($C_{13}$—$CH_3$, s), 1.40 ($C_{10}$—$CH_3$, s), 4.07 (C*$H_2$*Ø, dd), 5.61 ($C_{11}$—H, mult.), 6.09 ($C_4$—H, d), 6.21 ($C_2$—H, dd), 7.45 ($C_1$—H, d).

C. *9α-Bromo-17α-chloro-17β-[R]-benzylsulfinyl-11β-hydroxy-1,4-androstadiene-3-one*

Dissolve 0.36 gm. of 17α-chloro-17β-[R]-benzylsulfinyl-1,4,9(11)-androstatriene-3-one in 12 ml. of tetrahydrofuran, 1.32 ml. of water and 0.078 ml. of 70% aqueous perchloric acid. Add 0.156 gm. of N-bromosuccinimide and stir at 25°C. for 2 hours in the dark. Then add 1 ml. of 10% aqueous sodium metabisulfite solution and pour the total reaction mixture into water. Filter off the solids and wash with water and air dry. Recrystallize the solids from water/dimethylformamide to obtain the title product; nmr (dmso-$d_6$) δ 1.38 ($C_{13}$—$CH_3$, s), 1.65 ($C_{10}$—$CH_3$, s), 3.98 ($CH_2Ø$, s), 4.56 (11α—H, mult.), 5.91 ($C_4$—H, d), 6.05 ($C_2$—H, dd), 7.31 ($C_1$—H, d), 7.51 (Ø).

D. *9β,11β-Epoxy-17α-Chloro-17β-[R]-benzylsulfinyl-1,4-androstadiene-3-one*

Reflux 0.3 gm. of 9α-bromo-17α-chloro-17β-[R]-benzylsulfinyl-11β-hydroxy-1,4-androstadiene-3-one, 30 ml. of methanol, and 0.3 gm. of potassium acetate for 18 hours. Pour the solution into water, filter off the solids, wash with water and air dry to obtain the title compound; nmr ($CDCl_3$) δ 1.38 ($C_{13}$—$CH_3$, s), 1.40 ($C_{10}$—$CH_3$, s), 3.21 (11α—H, mult.) 3.96 ($CH_2Ø$, dd), 6.13 ($C_4$—H), 6.15 ($C_2$H, dd), 6.58 ($C_1$—H, d), 7.33 (Ø).

E. *9α-Fluoro-17α-chloro-17β-[R]-benzylsulfinyl-11β-hydroxy-1,4-androstadiene-3-one*

Dissolve 0.206 gm of 9β,11β-epoxy-17α-chloro-17β-[R]-benzylsulfinyl-1,4-androstadiene-3-one in 3.5 ml of 70% aqueous HF and stir at 0°C for 3 hours. Then pour the solution into a saturated $Na_2CO_3$ solution and extract with chloroform. Wash the chloroform extracts with water, dry over anhydrous sodium sulfate and then evaporate to a residue. Recrystallize the residue from methanol to obtain the title compound; m.p. 270°C (decomp.); nmr (dmso-$d_6$) δ 1.42 ($C_{13}$—$CH_3$, s), 1.54 ($C_{10}$—$CH_3$, s), 4.03 ($CH_2Ø$, dd), 4.20 (11α—H, mult.), 6.10 ($C_4$—H, d), 6.29 ($C_2$—H, dd), 7.33 ($C_1$—H, d). 7.44 (Ø).

F. *9α-Fluoro-17α-chloro-17β-[R]-benzylsulfinyl-1,4-androstadiene-3,11-dione*

Stir 50 mg of 9α-fluoro-17α-chloro-17β-[R]-benzylsulfinyl-11β-hydroxy-1,4-androstadiene-3-one in 5 ml of methylene chloride with 21 mg of sodium acetate and 27.5 mg of pyridinium chlorochromate for 2 hours at 25°C. Then filter through a small amount of silica gel and evaporate the filtrate to a residue. Recrystallize the residue from chloroform/hexane to obtain the title compound; m.p. 260°C (decomp.); nmr ($CDCl_3$) δ 1.20 ($C_{13}$—$CH_3$, s), 1.54 ($C_{10}$—$CH_3$, s), 4.08 ($CH_2Ø$, dd), 6.12 ($C_4$—H, d), 6.33 ($C_2$—H, dd), 7.40 (Ø), 7.43 ($C_1$—H, d).

G. *17α-Chloro-17β-benzylsulfonyl-1,4-androstadiene-3,11-dione*

Stir 45 mg of 17α-chloro-17β-[R]-benzylsulfinyl-1,4-androstadiene-3,11-dione in 5 ml of benzene with 45 mg of metachloroperbenzoic acid at 25°C for 1 hour. Dilute the reaction mixture with ethyl acetate and wash with 10% sodium metabisulfite solution and then with water. Dry the organic layer over anhydrous sodium sulfate, then evaporate to a residue. Recrystallize the residue from ethyl acetate/hexane to obtain the title compound; [$M^+$] 472, 474; [M—$PhCH_2SO_2^+$] 317, 319.

Acne is a common inflammatory disease in areas of the skin where sebaceous glands are largest, most numerous and most active. It is characterized by comedones, pustles, papules, inflamed nodules and, in extreme cases, infected sacs.

In acne patients sebum levels are evaluated. Therefore, it has been postulated that sebum, which is a secretion of the sebaceous gland, is a causative agent in acne. Indeed, when sebum levels are reduced in acne patients, the acne ameliorates. Some hormones used in anti-acne therapy reduce sebaceous gland activity but they usually demonstrate hormonal side effects.

Adolescents in puberty show a marked increase in hormonal production and are very likely to suffer from acne, since androgen, a hormone produced in puberty, has a great stimulatory effect on the sebaceous gland production of sebum.

Ideally, an anti-acne agent should be non-irritating and applicable topically or intralesionally, thereby avoiding the irritant effects of treatment with Vitamin A acid (retinoic acid). An anti-acne agent can also be administered orally if it does not have long-term systemic effects such as those of tetracycline or of hormones. The compounds of the present invention are useful anti-acne agents with good anti-sebaceous gland activity but with low or negligible systemic side effects (such as hormonal side effects) and with little or no irritation.

Decrease of lipogenesis in the flank organ of the hamster is the animal test model for the reduction of sebum production by the sebaceous gland (Lutsky *et al.* in *Journal of Investigative Dermatology,* Vol. 64, pages 412—417 (1975)). The compounds of this invention, as illustrated by formulae IA, II and III, in particular by formula II, most especially by 17α-chloro-17β-[R]-benzylsulfinyl-1,4-androstadiene-3,11-dione, reduce lipogenesis in the hamster flank organ and therefore demonstrate anti-sebaceous gland activity.

Additionally, excessive sebum production is a causative factor in seborrheic dermatitis, otherwise known as "oily" skin. On this basis, the compounds of the formula IA should be useful in combatting this condition.

The invention therefore provides pharmaceutical compositions, especially for use in the treatment of acne, comprising as active ingredient at least one steroid of the formula 1A defined above together with a pharmaceutically acceptable carrier. Such compositions can be prepared by bringing a steroid of the formula IA into a form suitable for therapeutic administration, e.g. by admixing it with a pharmaceutically acceptable carrier. Thus the compounds according to the invention can be used in a method of treating acne which comprises administering to a person affected by acne an effective amount of a steroid of the formula IA.

Thus, a composition comprising as active ingredient a steroid of the formula IA, preferably at a concentration in the range of from about 1% to about 20%, more preferably from about 2% to about 10% (by weight), together with a non-toxic, pharmaceutically acceptable carrier, is applied for example 2 to 5 times daily to skin affected by acne vulgaris, usually by the topical route—though intralesional injections may be in cases of severe acne—until the acne condition has improved. Alternatively, the combination can be administered orally or rectally. Such compositions are preferably in the form of dosage units, e.g. capsules, tablets or suppositories, which may contain for example from 10 to 200 mg of active ingredient of the formula IA per dosage unit.

The steroid of the formula IA is conveniently administered topically in a liquid solvent, the compositions being in the form of creams, lotions, solutions, gels suspensions, aerosols, ointments or intralesional injections.

The pharmaceutical compositions are made according to known procedures.

In the following Formulation Examples, the active ingredient named can if necessary be replaced by an equivalent quantity of another active ingredient of the Formula IA:

## Formulation Examples

### *Formulation 1: Cream (5%)*

| | mg/g |
|---|---|
| 17α-Chloro-17β-[R]-benzylsulfinyl-1,4-androstadiene-3,11-dione | 50.0 |
| White petrolatum, USP | 150.0 |
| Mineral oil, USP | 60.0 |
| Cetylstearyl alcohol | 72.0 |
| Cetomacrogol 1000 | 22.5 |
| 4-Chloro-*m*-cresol | 1.0 |
| Purified water USP to make | 1.0 g |

### *Formulation 2: Ointment (5%)*

| | mg/g |
|---|---|
| 17α-Chloro-17β-[R]-benzylsulfinyl-1,4-androstadiene-3,11-dione | 50.0 |
| Mineral oil, USP | 50.0 |
| Propylene glycol, USP | 50.0 |
| Petrolatum USP to make | 1.0 g |

*Formulation 3: Solution (5%)*

| | mg/ml |
|---|---|
| 17α-Chloro-17β-[R]-benzylsulfinyl-1,4-androstadiene-3,11-dione | 50.0 |
| Alcohol, USP | 50.0 |
| Propylene glycol, USP to make | 1.0 ml |

*Formulation 4: Lotion (5%)*

| | mg/ml |
|---|---|
| 17α-Chloro-17β-[R]-benzylsulfinyl-1,4-androstadiene-3,11-dione | 50.0 |
| Isopropyl alcohol NF | 500.0 |
| Carbopol 934 | 3.0 |
| Sodium hydroxide q.s. | |
| Purified water to make | 1.0 ml |

*Formulation 5: Gel (5%)*

| | mg/g |
|---|---|
| 17α-Chloro-17β-[R]-benzylsulfinyl-1,4-androstadiene-3,11-dione | 50.0 |
| Alcohol, USP | 150.0 |
| Carbopol 940 | 30.0 |
| Propylene glycol, USP | 150.0 |
| Diisopropanolamine (sufficient) | |
| Purified water, USP to make | 1.0 g |

*Formulation 6 Intralesional injection (5%)*

| | mg/ml |
|---|---|
| 17α-Chloro-17β-[R]-benzylsulfinyl-1,4-androstadiene-3,11-dione (sterile precipitated) | 50.0 |
| Monobasic sodium phosphate | 6.0 |
| Dibasic sodium phosphate anhydrous | 0.5 |
| Polysorbate 80, USP | 0.1 |
| Benzyl alcohol, R | 9.0 |
| Sodium chloride, USP | 2.5 |
| Methyl *p*-hydroxybenzoate, USP | 1.3 |
| Propyl *p*-hydroxybenzoate, USP | 0.2 |
| Sodium carboxymethylcellulose, USP | 3.0 |
| Disodium edetate, USP | 0.1 |
| Water for injection, USP g.s. ad | 1.0 ml |

*Formulation 7: Tablet (50 mg)*

| | | mg/Tablet |
|---|---|---|
| 17α-Chloro-17β-[R]-benzylsulfinyl-1,4-androstadiene-3,11-dione (micronized) | | 50 |
| Lactose | | 298 |
| Corn starch | | 25 |
| Corn starch paste (10% in water) | | 25 |
| Magnesium stearate | | 2 |
| | Total | 400 mg |

*Formulation 8: Capsule (50 mg)*

| | | mg/Capsule |
|---|---|---|
| 17α-Chloro-17β-[R]-benzylsulfinyl-1,4-androstadiene-3,11-dione (micronized) | | 50 |
| Lactose | | 298 |
| Corn starch | | 50 |
| Magnesium stearate | | 2 |
| | Total | 400 mg |

Encapsulate in two-piece hard gelatin capsules.

In this test-model, the flank gland of female hamsters is stimulated in growth with testosterone propionate (tp). A compound having anti-sebacious gland activity inhibits this growth.

Test compound: 17α-Chloro-17β-[R]-benzylsulfinyl-1,4-androstadiene-3,11-dione (Compound A).

Female hamsters weighing 100—110 g were housed singly and allowed food and water *ad libitum.* TP in sesame oil was injected s.c. daily; Compound A was dissolved in chloroform and applied topically to the left flank gland; the right flank gland received only chloroform; treatment lasted 14 days (excluding week-ends). The flank glands were excised and weighed at necropsy the day following the last treatment.

The Table shows that tp increased the weight of the flank glands approximately ten-fold over untreated controls, but the application of Compound A caused a smaller increase: Compound A at 0.1 mg produced a significantly smaller increase in the left flank gland without significantly affecting the weight of the contralateral, untreated flank gland; and at 0.5 mg and 1.0 mg, Compound A produced a significantly smaller increase in both treated and untreated flank glands, indicating a systemic effect.

ANTISEBACEOUS GLAND POTENCY OF COMPOUND A ON FLANK GLANDS OF TP–TREATED FEMALE HAMSTERS

| Daily Treatment[2] (mg) | Flank Gland Weight (mg ± S.E.) | |
|---|---|---|
| | Left Flank[3] (Treated) | Right Flank[3] (Untreated) |
| Controls | 3.03 ± 0.27** | 2.86 ± 0.24** |
| TP – 0.125 | 32.55 ± 0.50 | 31.08 ± 0.83 |
| TP – 0.125 + Compound A – 0.1 | 23.12 ± 1.62** (32) | 27.15 ± 2.06 (14) |
| TP – 0.125 + Compound A – 0.5 | 19.88 ± 1.58** (43) | 23.40 ± 1.57** (27) |
| TP – 0.125 + Compound A – 1.0 | 17.12 ± 2.49** (52) | 21.05 ± 1.83** (35) |

[1] Six animals/group. TP = testosterone propionate. S.E. = standard error.

[2] TP dissolved in sesame oil and injected s.c., Compound A dissolved in chloroform and applied only to left flank daily for 14 days (weekends excluded).

[3] Numbers in parentheses are per cent inhibition.

** Significantly different from TP-treatment hamsters $P < 0.01$.

CLAIMS:

1. A steroid of the formula

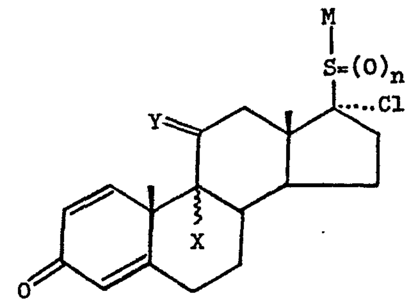

I

wherein M is a benzyl, phenethyl, methylbenzyl, dimethylbenzyl, chlorobenzyl or dichlorobenzyl group or an alkyl group having from 1 to 8 carbon atoms,
X is an $\alpha$-hydrogen, $\alpha$-fluorine or $\alpha$-bromine atom,
Y is (H,$\beta$—OH), or, provided that X is an $\alpha$-hydrogen or $\alpha$-fluorine atom, Y can also be an oxygen atom, or, provided that X is an $\alpha$-hydrogen atom, Y can also be (H,H), or X and Y together are a further 9,11 bond or a 9$\beta$,11$\beta$-epoxy group, together with an 11-hydrogen atom,
and $n$ is 1, or, when Y is an oxygen atom or (H,H), $n$ can also be 2.

2. A steroid as claimed in claim 1 having the formula:

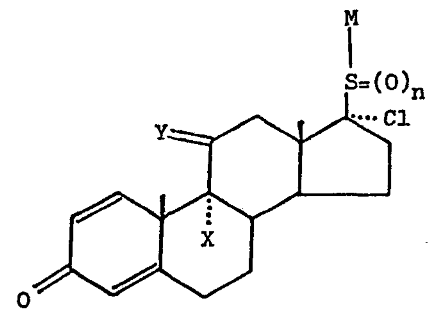

IA

wherein M is as defined in claim 1,
X is an $\alpha$-hydrogen or $\alpha$-fluorine atom,
Y is an oxygen atom, or, provided that X is an $\alpha$-hydrogen atom, Y can also be (H,H),
and $n$ is 1 or 2,
preferably a steroid of the formula

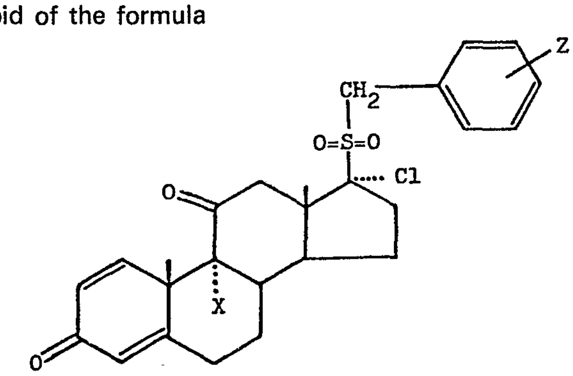

III

wherein X is a fluorine or hydrogen atom and Z is a hydrogen atom, one or two methyl groups or one or two chlorine atoms.

3. A steroid as claimed in claim 1 having the formula:

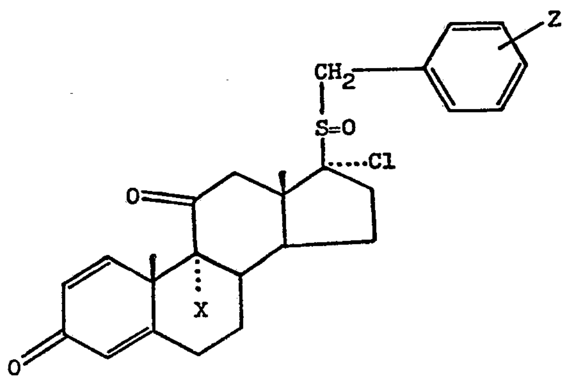

II

wherein X and Z are as defined in claim 2, preferably
17$\alpha$-Chloro-17$\beta$-[R]-benzylsulfinyl-1,4-androstadiene-3,11-dione,
17$\alpha$-Chloro-17$\beta$-(4-chlorobenzylsulfinyl)-1,4-androstadiene-3,11-dione,
17$\alpha$-Chloro-17$\beta$-(2,4-dichlorobenzylsulfinyl)-1,4-androstadiene-3,11-dione,
17$\alpha$-Chloro-17$\beta$-(3,4-dichlorobenzylsulfinyl)-1,4-androstadiene-3,11-dione,
17$\alpha$-Chloro-17$\beta$-(2-methylbenzylsulfinyl)-1,4-androstadiene-3,11-dione, and
9$\alpha$-Fluoro-17$\alpha$-chloro-17$\beta$-[R]-benzylsulfinyl-1,4-androstadiene-3,11-dione.

4. A steroid as claimed in claim 1 having the formula

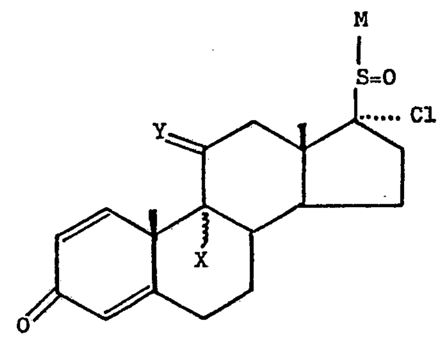

IC

wherein M is as defined in claim 1,
X is an $\alpha$-hydrogen, $\alpha$-fluorine or $\alpha$-bromine atom, and Y is (H,$\beta$—OH),
or X and Y together are a further 9,11-bond or a 9$\beta$,11$\beta$-epoxy group, together with an 11-hydrogen atom.

5. A process for the preparation of a 17$\alpha$-chloro-17$\beta$-(M-sulfinyl)-1,4-androstadiene-3-one having the following partial formula at C—17

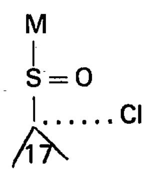

wherein M is as defined above, which comprises reacting a 17$\alpha$-(M-thio)-1,4-androstadiene-3-one having the following partial formula at C—17

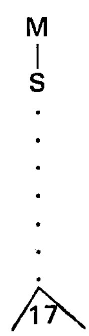

wherein M is as defined above, with a chlorine source in a solvent system comprising water and a water-soluble base, at a temperature from about −78°C to about 25°C, preferably with 2 to 5 equivalents of chlorine gas, N-chlorosuccinimide, 1-chlorobenzotriazole, or iodobenzene dichloride as chlorine source at a temperature from about −40°C to about 0°C.

6. A process as claimed in claim 5 wherein at least 3 equivalents of a basic water-miscible organic solvent selected from pyridine, collidine and triethylamine is present in the solvent system.

7. A process as claimed in claim 5 wherein a steroid of the formula

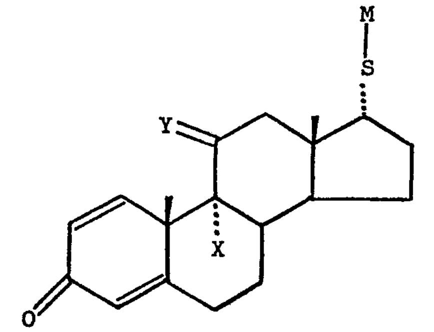

IV

wherein M is as defined in claim 1,
X is a hydrogen or fluorine atom,
and Y is an oxygen atom, or, provided that X is an α-hydrogen atom, Y can also be (H,H), is allowed to react with about 3 equivalents of iodobenzene dichloride in aqueous pyridine at about −40°C to produce a compound of the formula

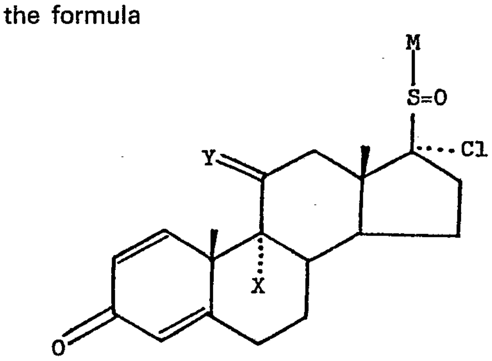

IB

wherein M, X and Y are as defined for formula IV.

8. A process for the preparation of a compound of the formula IA in claim 2 wherein X is a fluorine atom, Y is an oxygen atom and *n* is 1, which comprises introducing a 9α-fluorine atom by the following sequence of reactions a) to f) into a compound of the formula IA prepared by a process as claimed in any of claims 5 to 7 and having a 9α-hydrogen atom:

a) Reduction of the 11-keto group to 11β-hydroxy;
b) Dehydration to introduce a 9(11)-double bond;
c) Reaction of the 1,4,9(11)-androstatriene from step b) with a bromonium source and a strong acid in an aqueous organic solvent to yield the corresponding 9α-bromo-11β-hydroxy-1,4-androstadiene;
d) 9,11-Dehydrohalogenation to yield the 9β,11β-epoxide;
e) Reaction of this epoxide with hydrofluoric acid to yield the corresponding 9α-fluoro-11β-hydroxy-1,4 androstadiene;
f) Oxidation at the 11-position to yield the desired compound of the formula IA wherein X is a fluorine atom and Y is an oxygen atom;

preferably a process wherein

a) Reduction of the 11-keto group is effected with sodium borohydride in an organic solvent comprising methanol or dimethylformamide;
b) The dehydration is effected with methanesulfonyl chloride and collidine in dimethylformamide in the presence of sulfur dioxide;
c) The bromonium source is *N*-bromo-succinimide, the strong acid is perchloric acid, and the aqueous organic solvent is moist dioxan or tetrahydrofuran;
d) The dehydrohalogenation is effected by means of potassium acetate in methanol;
e) The hydrofluroic acid is about 70% hydrofluroic acid;
f) The oxidation is effected with pyridinium chlorochromate in methylene chloride.

9. A process as claimed in any of claims 5 to 8 wherein a compound of the formula IA wherein *n* is 1 is oxidized to a compound of the formula IA wherein *n* is 2, preferably with *m*-chloroperbenzoic acid in an inert organic solvent.

10. Pharmaceutical compositions, especially for use in the treatment of acne, comprising as active ingredient at least one compound of the formula IA defined in claim 2 together with a pharmaceutically acceptable carrier.

11. Compositions as claimed in claim 10 in the form of creams, lotions, ointments, aerosols, solutions, gels or suspensions, or adapted for intralesional injection, and preferably containing from 1% to 20% of active ingredient; or in the form of tablets, capsules or suppositories, preferably containing from 10 to 200 mg of active ingredient.

**Revendications**

1. Stéroïde, caractérisé en ce qu'il a la formule suivante:

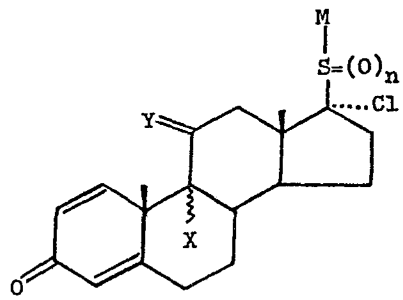

I

dans laquelle M représente un groupe benzyle, phénéthyle, méthylbenzyle, diméthylbenzyle, chlorobenzyle ou dichlorobenzyle ou un groupe alcoyle ayant de 1 à 8 atomes de carbone,
X représente un atome d'hydrogène$\alpha$-, de fluor$\alpha$- ou de brome$\alpha$-,
Y représente (H, $\beta$—OH), ou, lorsque X est une atome d'hydrogène$\alpha$- ou de fluor$\alpha$-, Y peut également représenter un atome d'oxygène, ou, lorsque X représente un atome d'hydrogène$\alpha$-, Y peut également représenter (H,H), ou X et Y ensemble représentent une liaison 9,11-supplémentaire ou un groupe 9$\beta$, 11$\beta$-époxy, ensemble avec un atome d'hydrogène en position 11,
et *n* est égal à 1, ou, lorsque Y représente un atome d'oxygène ou (H,H), *n* peut également être égal à 2.

2. Stéroïde selon la revendication 1, caractérise en ce qu'il a la formule suivante:

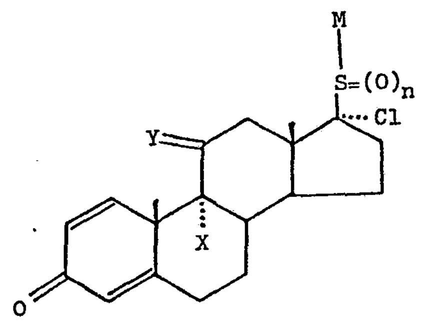

IA

dans laquelle M est tel que défini à la revendication 1,
X représente un atome d'hydrogène$\alpha$- ou de fluor $\alpha$-,
Y représente un atome d'oxygène, ou, lorsque X représente un atome d'hydrogène$\alpha$-, X peut également représenter (H,H) et *n* est égal à 1 ou 2,
de préférence un stéroïde de la formule:

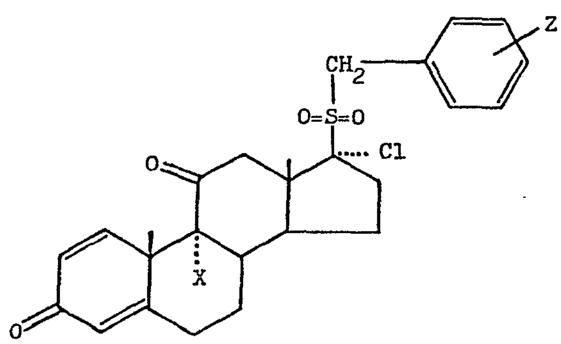

III

dans laquelle X représente un atome de fluor ou d'hydrogène et Z représente un atome d'hydrogène, un ou deux groupes méthyle ou un ou deux atomes de chlore.

3. Stéroïde selon la revendication 1, caractérisé en ce qu'il a la formule suivante:

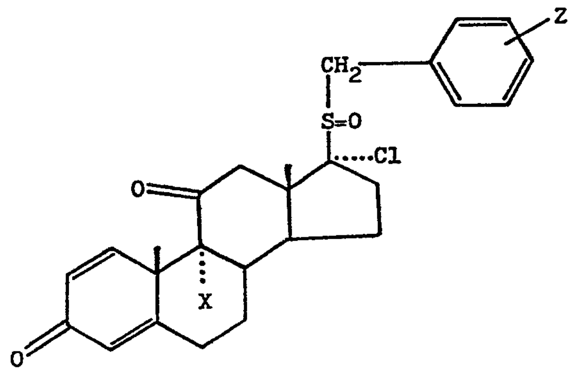

II

dans laquelle X et Z sont tels que définis à la revendication 2, de préférence
17α-chloro-17β-[R]-benzylsulfinyl-1,4-androstadiène-3,11-dione,
17α-chloro-17β-(4-chlorobenzylsulfinyl)-1,4-androstadiène-3,11-dione,
17α-chloro-17β-(2,4-dichlorobenzylsulfinyl)-1,4-androstadiène-3,11-dione,
17α-chloro-17β-(3,4-dichlorobenzylsulfinyl)-1,4-androstadiène-3,11-dione,
17α-chloro-17β-(2-méthylbenzylsulfinyl)-1,4-androstadiène-3,11-dione et
9α-fluoro-17α-chloro-17β-[R]-benzylsulfinyl-1,4-androstadiène-3,11-dione,

4. Stéroïde selon la revendication 1, caractérisé en ce qu'il a la formule suivante:

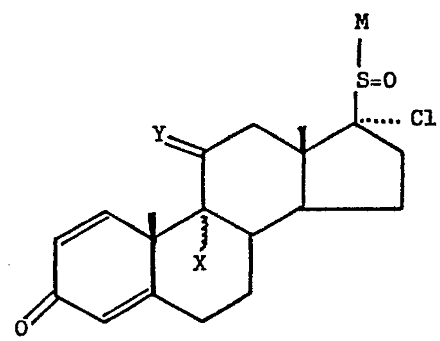

IC

dans laquelle M est tel que défini à la revendication 1, X représente un atome d'hydrogèneα-, de fluorα- ou de bromeα-,
et Y représente (H,β—OH),
ou X et Y ensemble représentent une liaison 9,11-supplémentaire ou un groupe 9β,11β-époxy, ensemble avec un atome d'hydrogène en position 11.

5. Procédé de préparation d'une 17α-chloro-17β-(M-sulfinyl)-1,4 androstadiène-3-one ayant la formule partielle suivante en C—17

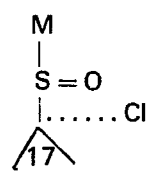

dans laquelle M est tel que défini à la revendication 1, caractérisé en ce qu'il comprend la mise en réaction d'une 17α-(M-thio)-1,4-androstadiène-3-one ayant la formule partielle suivante en C—17

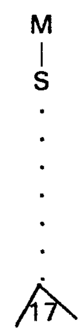

dans laquelle M est tel que défini à la revendication 1, avec une source de chlore dans un système de solvant comprenant de l'eau et une base soluble dans l'eau, à une température comprise entre environ —78°C et environ 25°C, de préférence avec 2 à 5 équivalents de chlore gazeux, du N-chlorosuccinimide, du 1-chlorobenzotriazole, ou du dichlorure d'iodobenzène comme source de chlore à une température comprise entre environ —40°C et environ 0°C.

6. Procédé selon la revendication 5, caractérisé en ce qu'au moins 3 équivalents d'un solvant organique miscible à l'eau basique, choisis parmi la pyridine, la collidine et la triéthylamine est présent dans le système de solvant.

7. Procédé selon la revendication 5, caractérisé en ce qu'on met à réagir un stéroïde de la formule suivante:

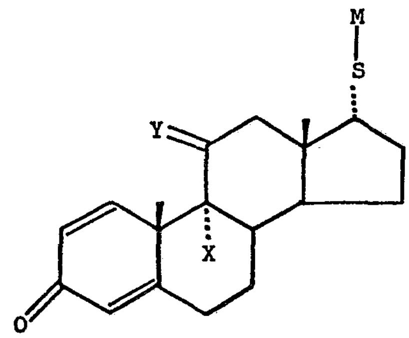

IV

dans laquelle M est tel que défini à la revendication 1, X représente un atome d'hydrogène$\alpha$- ou un atome de fluor$\alpha$-,
et Y représente un atome d'oxygène, ou, lorsque X représente un atome d'hydrogène$\alpha$-, Y peut également représenter (H,H),
avec environ 3 équivalents de dichlorure d'iodobenzène dans de la pyridine aqueuse à environ —40°C pour produire un composé de la formule suivante:

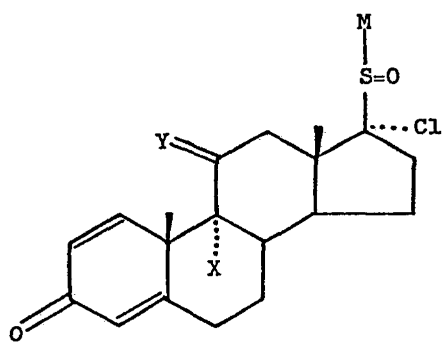

IB

dans laquelle M, X et Y sont tels que définis pour la formule IV.

8. Procédé de préparation d'un composé de la formule IA selon la revendication 2, dans laquelle X représente un atome de fluor, et Y représente un atome d'oxygène et *n* est égal à 1, caractérisé en ce qu'il comprend l'introduction d'un atome 9$\alpha$-fluor par la séquence suivante de réaction a) à f) dans un composé de la formule IA préparé par un procédé selon l'une quelconque des revendications 5 à 7 et ayant un atome 9$\alpha$-hydrogène;

a) La réduction du groupe 11-céto en 11$\beta$-hydroxy;
b) La déshydratation pour introduire une double liaison 9(11)-;
c) La réaction du 1,4,9(11)-androstatriène à partir de l'étape b) avec une source de bromonium et un acide fort dans un solvant organique aqueux pour donner le 9$\alpha$-bromo-11$\beta$-hydroxy-1,4-androstadiène correspondant;
d) La 9,11-déshydrohalogénation pour donner le 9$\beta$, 11$\beta$-époxyde;
e) La réaction de cet époxyde avec l'acide fluorhydrique pour donner le 9$\alpha$-fluoro-11$\beta$-hyroxy-1,4-androstadiène correspondant;
f) L'oxydation à la position 11 pour obtenir le composé désiré de la formule IA dans laquelle X présente un atome de fluor et Y représente un atome d'oxygène;

de préférence un procédé dans lequel:

a) La réduction du groupe 11-céto est effectuée avec du borohydrure de sodium dans un solvant organique comprenant le méthanol ou le diméthylformamide;
b) La déshydratation est effectuée avec la chlorure de méthanesulfonyle et la collidine dans du diméthylformamide en présence de dioxyde de soufre;
c) La source de bromonium est le N-bromo-succinimide, l'acide fort est l'acide perchlorique et le solvant organique aqueux est du dioxane ou du tétrahydrofuranne humide;
d) La déshydrohalogénation est effectuée au moyen d'acétate de potassium dans du méthanol;

e) L'acide fluorhydrique est de l'acide fluorhydrique à environ 70%;
f) L'oxydation est effectuée avec du chlorochromate de pyridinium dans du chlorure de méthylène.

9. Procédé selon l'une quelconque des revendications 5 à 8, caractérisé en ce qu'un composé de la formule IA dans laquelle *n* est égal à 1 est oxydé en un composé de la formule IA dans laquelle *n* est égal à 2, de préférence avec de l'acide *m*-chloroperbenzoïque dans un solvant organique inerte.

10. Compositions pharmaceutiques, spécialement pour l'emploi dans le traitement de l'acné, caracterisées en ce qu'elles comprennent comme ingrédient actif au moins un composé de la formule IA définie à la revendication 2, avec un support pharmaceutiquement acceptable.

11. Compositions selon la revendication 10, caractérisées en ce qu'elles se trouvent sous la forme de crèmes, lotions, onguents, aérosols, solutions, gels ou suspensions, ou sous une forme adaptée pour l'injection intra-lésionnelle, et de préférence contenant de 1% à 20% d'un ingrédient actif; ou sous la forme de comprimés, capsules ou suppositoires, de préféfence contenant de 10 à 200 mg d'ingrédient actif.

**Patentansprüche**

1. Ein Steroid der Formel

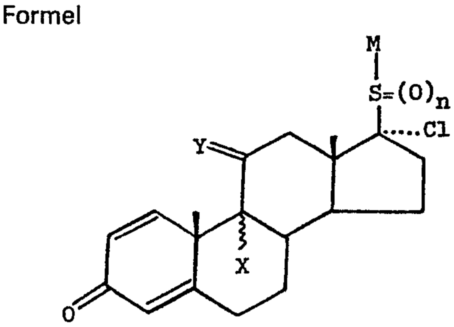

I

in der M ein Benzyl-, Phenethyl-, Methylbenzyl-, Dimethylbenzyl-, Chlorbenzyl- oder Dichlorbenzylrest oder ein Alkylrest mit 1 bis 8 C-Atomen ist,
X ein $\alpha$-Wasserstoff-, $\alpha$-Fluor- oder $\alpha$-Bromatom ist,
Y für (H,$\beta$—OH) steht oder, wenn X ein $\alpha$-Wasserstoff oder $\alpha$-Fluoratom ist, auch ein Sauerstoffatom oder, wenn X ein $\alpha$-Wasserstoffatom ist, auch (H,H) sein kann oder X und Y gemeinsam eine weitere 9,11-Bindung oder eine 9$\beta$,11$\beta$-Epoxygruppe zusammen mit einem 11-Wasserstoffatom bilden, und n für 1 steht oder, wenn Y ein Sauerstoffatom oder (H,H) ist, auch 2 sein kann.

2. Steroid nach Anspruch 1 mit der Formel

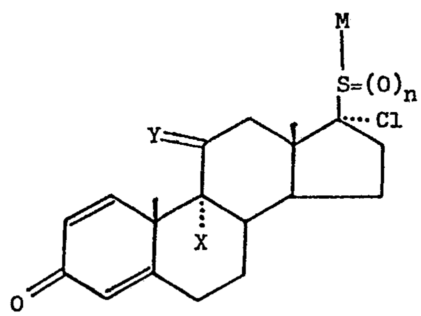

IA

in der M die in Anspruch 1 genannte Bedeutung hat,
X ein $\alpha$-Wasserstoff- oder $\alpha$-Fluoratom,
Y ein Sauerstoffatom ist oder, wenn X ein $\alpha$-Wasserstoffatom ist, auch (H,H) sein kann und n für 1 oder 2 steht, vorzugsweise ein Steroid der Formel

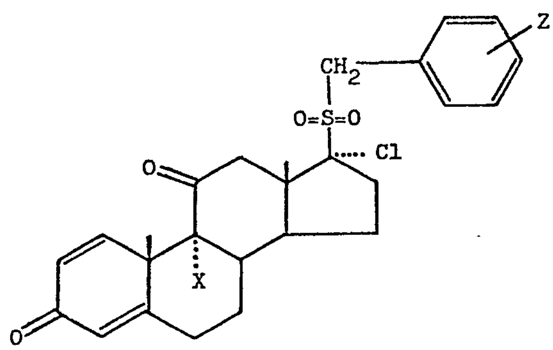

III

in der X ein Fluor- oder Wasserstoffatom und Z ein Wasserstoffatom, eine oder zwei Methylreste oder ein oder zwei Chloratome ist.

3. Steroid nach Anspruch 1 mit der Formel

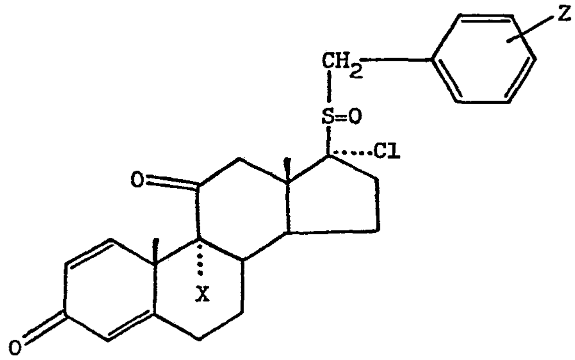

II

in der X und Z die in Anspruch 2 genannten Bedeutungen haben, vorzugsweise
17$\alpha$-Chlor-17$\beta$-[R]-benzylsulfinyl-1,4-androstadien-3,11-dion,
17$\alpha$-Chlor-17$\beta$-(4-chlorbenzylsulfinyl)-1,4-androstadien-3,11-dion,
17$\alpha$-Chlor-17$\beta$-(2,4-dichlorbenzylsulfinyl)-1,4-androstadien-3,11-dion,
17$\alpha$-Chlor-17$\beta$-(3,4-dichlorbenzylsulfinyl)-1,4-androstadien-3,11-dion,
17$\alpha$-Chlor-17$\beta$-(2-methyl-benzylsulfinyl)-1,4-androstadien-3,11-dion und
9$\alpha$-Fluor-17$\alpha$-chlor-17$\beta$-[R]-benzylsulfinyl-1,4-androstadien-3,11-dion,

4. Steroid nach Anspruch 1 mit der Formel

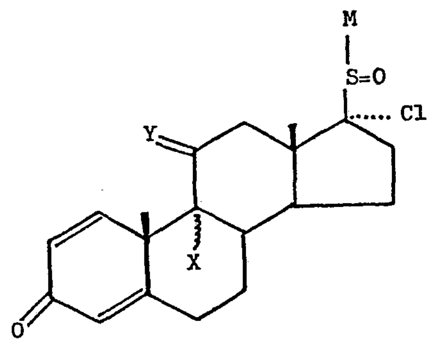

IC

in der M die in Anspruch 1 genannte Bedeutung hat, X ein $\alpha$-Wasserstoff-, $\alpha$-Fluor- oder $\alpha$-Bromatom ist und Y für (H,$\beta$—OH) steht oder X und Y zusammen eine weitere 9,11-Bindung ode eine 9$\beta$,11$\beta$-Epoxygruppe zusammen mit einem 11-Wasserstoffatom sind.

5. Verfahren zur Herstellung eines 17$\alpha$-Chlor-17$\beta$-(M-sulfinyl)-1,4-androstadien-3-ons der folgenden partiellen Formel bei C—17

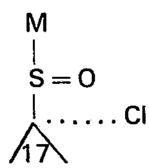

in der M die in Anspruch 1 genannte Bedeutung hat, dadurch gekennzeichnet, daß man ein 17$\alpha$-(M-Thio)-1,4-androstadien-3-on der folgenden partiellen Formel bei $C_{17}$

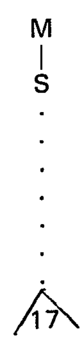

in der M die in Anspruch 1 genannte Bedeutung hat, mit einer Chlorquelle in einem Wasser und eine wasserlösliche Base enthaltenden Lösungsmittelsystem bei einer Temperatur von etwa —78° bis etwa 25°C, vorzugsweise mit 2 bis 5 Äquivalenten Chlorgas, N-chlorsuccinimid, 1-Chlorbenzotriazol oder Jodbenzoldichlorid als Chlorquelle bei einer Temperatur von etwa —40°C bis etwa 0°C umsetzt.

6. Verfahren nach Anspruch 5, dadurch gekennzeichnet, daß wenigstens 3 Äquivalente eines basischen, mit Wasser mischbaren organischen Lösungsmittel, das aus Pyridin, Kollidin und Triäthylamin ausgewählt ist, im Lösungsmittelsystem vorhanden sind.

7. Verfahren nach Anspruch 5, dadurch gekennzeichnet, daß man ein Steroid der Formel

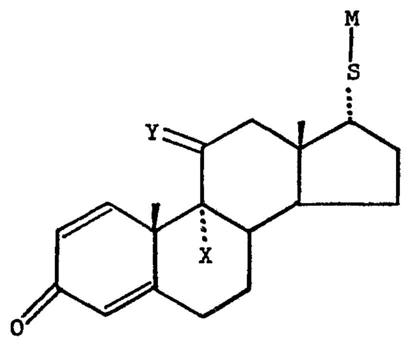

IV

in der M die in Anspruch 1 genannte Bedeutung hat, X ein $\alpha$-Wasserstoffatom oder $\alpha$-Fluoratom und Y ein Sauerstoffatom ist oder, wenn X ein $\alpha$-Wasserstoffatom ist, auch (H,H) sein kann, mit etwa 3 Äquivalenten Jodbenzoldichlorid in wäßrigem Pyridin bei etwa —40°C zu einer Verbindung der Formel

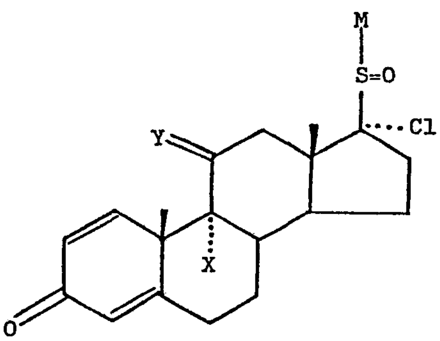

IB

umsetzt, in der M, X und Y die für Formel IV genannten Bedeutungen haben.

8. Verfahren zur Herstellung einer Verbindung der Formel IA in Anspruch 2, in der X ein Fluoratom, Y ein Sauerstoffatom ist und n für 1 steht, dadurch gekennzeichnet, daß man ein 9$\alpha$-Fluoratom durch die folgende Aufeinanderfolge von Reaktionen (a) bis (f) in ein nach dem Verfahren gemäß Anspruch 5 bis 7 hergestellte und ein 9$\alpha$-Wasserstoffatom enthaltende Verbindung der Formel IA einführt:

a) Reduktion der 11-Ketogruppe zu 11$\beta$-Hydroxy,
b) Dehydratisierung zur Einführung einer 9(11)-Doppelbindung,
c) Umsetzung des 1,4,9(11)-Androstatriens aus Stufe (b)mit einer Bromoniumquelle und einer starken Säure in einem wäßrigen organischen Lösungsmittel unter Bildung des entsprechenden 9$\alpha$-Brom-11$\beta$-hydroxy-1,4-androstadiens,
d) 9,11-Dehydrohalogenierung unter Bildung des 9$\beta$,11$\beta$ Epoxids,
e) Umsetzung dieses Epoxids mit Fluorwasserstoffsäure unter Bildung des entsprechenden 9$\alpha$-Fluor-11$\beta$-hydroxy-1,4-androstadiens,
f) Oxidation an der 11-Stellung unter Bildung der gewünschten Verbindung der Formel IA, in der X ein Fluoratom und Y ein Sauerstoffatom ist,

Vorzugsweise ein Verfahren, bei dem

a) die Reduktion der 11-Ketogruppe mit Natriumborhydrid in einem Methanol oder Dimethylformamid enthaltenden organischen Lösungsmittel durchgeführt wird,
b) die Dehydratisierung mit Methansulfonylchlorid und Kollidin in Dimethylformamid in Gegenwart von Schwefeldioxid durchgeführt wird,
c) die Bromoniumquelle M-Brom-succinimid ist, die starke Säure Perchlorsäure ist und das wäßrige organische Lösungsmittel feuchtes Dioxan oder Tetrahydrofuran ist,
d) die Dehydrohalogenierung mit Hilfe von Kaliumacetat in Methanol durchgeführt wird,
e) die Fluorwasserstoffsäure eine etwa 70%ige Fluorwasserstoffsäure ist und
f) die Oxidation mit Pyridiniumchlorchromat in Methylenchlorid durchgeführt wird.

9. Verfahren nach einem der Ansprüche 5 bis 8, dadurch gekennzeichnet, daß man eine Verbindung der Formel IA, in der n für 1 steht, zu einer Verbindung der Formel IA, in der n für 2 steht, vorzungsweise mit m-Chlorperbenzoesäure in einem inerten organischen Lösungsmittel oxidiert.

10. Arzneimittelzubereitungen, insbesondere für die Verwendung zur Behandlung der Akne, enthaltend als Wirkstoff wenigstens eine Verbindung der in Anspruch 2 definierten Formel IA zusammen mit einem pharmazeutisch unbedenklichen Trägfer.

11. Arzneimittelzubereitungen nach Anspruch 10, die in Form von Cremes, Lotionen, Salben, Aerosolen, Lösungen, Gelen oder Suspensionen Vorliegen oder für die intraläsionale Injektion angepaßt sind und vorzugsweise 1% bis 20% Wirkstoff enthalten oder in Form von Tabletten, Kapseln oder Suppositorien vorliegen, die vorzugsweise 10 bis 200 mg Wirkstoff enthalten.